# EUROPEAN PATENT APPLICATION

(11) **EP 1 813 301 A1**
(43) Date of publication of application: **01.08.2007**
(21) Application number: 06017007.3
(22) Date of filing: 16.08.2006
(51) Int. Cl.: A61M 5/32, A61M 5/50

(54) **Safety hypodermic syringe**

(30) Priority: 26.01.2006 CN 200610002659
(71) Applicant: Chen, Chang-Tzu, Taipei (TW)
(72) Inventor: Chen, Chang-Tzu, Taipei (TW)
(74) Representative: Helms, Joachim

(57) **Abstract**

A safety hypodermic syringe is disclosed to include a barrel, which has a front connector with a breakable connection portion connected to the peripheral wall of the barrel, a needle assembly connected to the front connector, a flexible stopper fitting the inner diameter of the barrel, a plunger, which has a stopper holder that holds the flexible stopper and a barbed retaining flange disposed at the front side for engaging two hollow retaining wings of the front connector when the plunger is pushed further forward after the service of the safety hypodermic syringe such that the front connector is separated from the barrel and received with the needle assembly to the inside of the barrel upon a return stroke of the plunger.

## Description

### BACKGROUND OF THE INVENTION

### Field of the Invention:

The present invention relates to a hypodermic syringe and more particularly, to a safety hypodermic syringe that prevents vibration of the needle assembly and leakage of the contained fluid medicine.

### Description of the Related Art:

A hypodermic syringe is an important medical instrument adapted for use to inject or withdraw liquid medicines. In recent years, the spreading of AIDS and many other diseases that are difficult to treat causes many contamination accidents. In order to prevent contamination, self-destructive safety hypodermic syringes are developed.

A safety hypodermic syringe is known comprising a needle assembly, a medicine barrel, a plunger, and an outer barrel. The needle assembly is fastened to the front side of the medicine barrel. The plunger is axially movably coupled to the medicine barrel and mounted with the medicine barrel inside the outer barrel. After the service of the hypodermic syringe, the plunger is pulled backwards to carry the needle cannula or the needle assembly backwards to the inside of the medicine barrel. This design of safety hypodermic syringe is functional, however it has a complicated structure and comprised of a big number of parts, resulting in a high manufacturing cost.

Taiwan Patent Publication No. 540385, issued on July 1st, 2003, discloses a safety hypodermic syringe entitled "Two Piece Type Retractable Safety Hypodermic Syringe". According to this design, the safety hypodermic syringe comprises a barrel, a plunger inserted into the barrel, a needle holder provided at the front side of the barrel to hold a needle, and a block with a through hole installed in the needle holder. The barrel has a tear groove on the front wall around the needle holder. After engagement of the head of the plunger with the needle holder, the plunger is pulled backwards to break the tear groove, causing the needle holder and the needle to be carried with the plunger backwards to the inside of the barrel. According to this design, the thin tear groove on the front wall of the barrel may be broken accidentally during injection or transportation of the safety hypodermic syringe, thereby causing a leakage of the fluid medicine.

Further, according to conventional designs, a residual amount of the applied liquid medicine will be left in the tubular front neck of the barrel and the hub of the needle assembly after the stopper has been pushed to the front limit position and stopped at the front wall of the barrel.

Therefore, it is desirable to provide a safety hypodermic syringe that eliminates the aforesaid drawbacks.

### SUMMARY OF THE INVENTION

The present invention has been accomplished under the circumstances in view. According to one embodiment of the present invention, the safety hypodermic syringe comprises a barrel, the barrel comprising a fluid chamber, and a front connector suspending in a front end thereof, the front connector defining an axially extending fluid passage in communication between the fluid chamber and the atmosphere, the front connector having two retaining portions protruded from the periphery thereof at two sides, a connection portion extending around the periphery of a rear end thereof and connected to the periphery of the barrel, and a tearing groove formed on the connection portion; a needle assembly, the needle assembly comprising a hub and a needle cannula forwardly extending from the hub, the hub having an axial hole disposed in fluid communication with the needle cannula and a rear coupling portion capped on the front connector of the barrel and engaged into the tearing groove; a plunger, the plunger comprising a shank, a front extension rod forwardly axially extending from a front end of the shank, and a retaining portion at the front extension rod; and a flexible stopper fitting the diameter of the fluid chamber of the barrel, the stopper having a longitudinal chamber through which the front extension rod of the plunger passes, and a front center through hole forwardly extending from the longitudinal chamber to a front side thereof and stopped in front of the retaining portion of the plunger; wherein when pushed the plunger forwards after the service of the safety hypodermic syringe, the retaining portion of the plunger is forced into engagement with the retaining portions of the front connector and the tearing groove is broken to separate the front connector from the barrel for enabling the broken front connector and the needle assembly to be pulled with the plunger backwards to the inside of the barrel upon a return stroke of the plunger at this time.

The retaining portions of the front connector are hollow retaining wings; the retaining portion of the plunger is a barbed retaining flange for engaging the hollow retaining wings of said front connector.

The coupling portion of the hub of the needle assembly has a beveled outer surface disposed in contact with the connection portion of the front connector of the barrel.

According to another embodiment of the present invention, the safety hypodermic syringe comprises a barrel, the barrel comprising a fluid chamber, and a front connector suspending in a front end thereof, the front connector defining an axially extending fluid passage in communication between the fluid chamber and the atmosphere, the front connector having two hollow retaining wings protruded from the periphery thereof at two sides, a connection portion extending around the periphery of a rear end thereof and connected to the periphery of the barrel, and a tearing groove formed on the connection portion; a needle assembly, the needle assembly comprising a hub and a needle cannula forwardly extending from the hub, the hub having an axial hole disposed in fluid communication with the needle cannula and connected to the front connector of the barrel; a plunger, the plunger comprising a shank, a front extension rod forwardly axially extending from a front end of the shank, and a barbed retaining flange around the periphery of a front end of the front extension rod; and a flexible stopper fitting the diameter of the fluid chamber of the barrel, the stopper having a longitudinal chamber through which the front extension rod of the plunger passes, and a front center through hole forwardly extending from the longitudinal chamber to a front side thereof and stopped in front of the barbed retaining flange of the plunger; wherein when pushed the plunger forwards after the service of the safety hypodermic syringe, the barbed retaining flange of the plunger is forced into engagement with the hollow retaining wings of the front connector and the tearing groove is broken to separate the front connector from the barrel for enabling the broken front connector and the needle assembly to be pulled with the plunger backwards to the inside of the barrel upon a return stroke of the plunger at this time.

The barrel further has an inside annular stop flange extending around an inside wall near a rear end thereof: the plunger has a head connected between the shank and the front extension rod, the head having a front flange and a rear flange for engagement with the inside annular stop flange of the barrel after a return stroke of the plunger after the service of the safety hypodermic syringe. The plunger further has a neck connected between the shank and the head for breaking to separate the shank from the head after engagement of the front flange and rear flange of the head with the inside annular stop flange of the barrel, and a stopper holder formed integral with the front extension rod for holding the stopper. The stopper holder has a front retaining skirt and a rear retaining skirt respectively extending around the periphery thereof; the flexible stopper having an inside annular flange formed in a rear side of the longitudinal chamber and disposed in contact with the periphery of the stopper holder between the front skirt and the rear skirt.

### BRIEF DESCRIPTION OF THE DRAWINGS

FIG. 1a is an exploded view of a safety hypodermic syringe according the present invention.
FIG. 1b corresponds to FIG. 1a when viewed from another angle.
FIG. 2 is a perspective assembly view of the safety hypodermic syringe according to the present invention.
FIG. 3a is a top view in an enlarged scale of the safety hypodermic syringe according to the present invention.
FIG. 3b is a sectional view in an enlarged scale of the front part of the barrel of the safety hypodermic syringe according to the present invention.
FIG. 4 is a sectional view of the present invention, showing a standby status of the safety hypodermic syringe.
FIG. 5 is a schematic sectional view of the present invention, showing the barrel of the safety hypodermic syringe filled with a liquid medicine for injection.
FIG. 6 is a schematic sectional view of the present invention, showing the status of the hypodermic syringe after injection.
FIG. 7 is a schematic sectional enlarged view of a part of the present invention, showing the barbed retaining flange of the plunger engaged with the retaining wings of the conical front connector of the barrel.
FIG. 8 is a schematic sectional enlarged view of a part of the present invention, showing the conical front connector disconnected from the peripheral wall of the barrel.
FIG. 9 is a schematic sectional enlarged view of the present invention, showing the needle assembly received inside the barrel after a return stroke of the plunger.
FIG. 10 is a schematic sectional enlarged view of the present invention, showing the neck of the plunger broken, the needle assembly with the front part of the plunger left inside the barrel.

### DETAILED DESCRIPTION OF THE PREFERRED EMBODIMENT

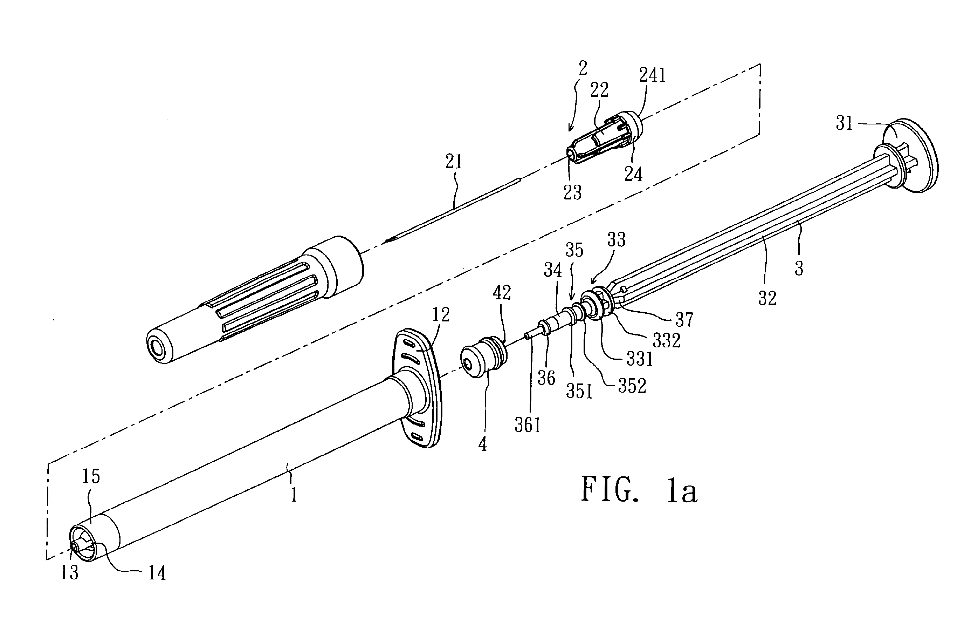

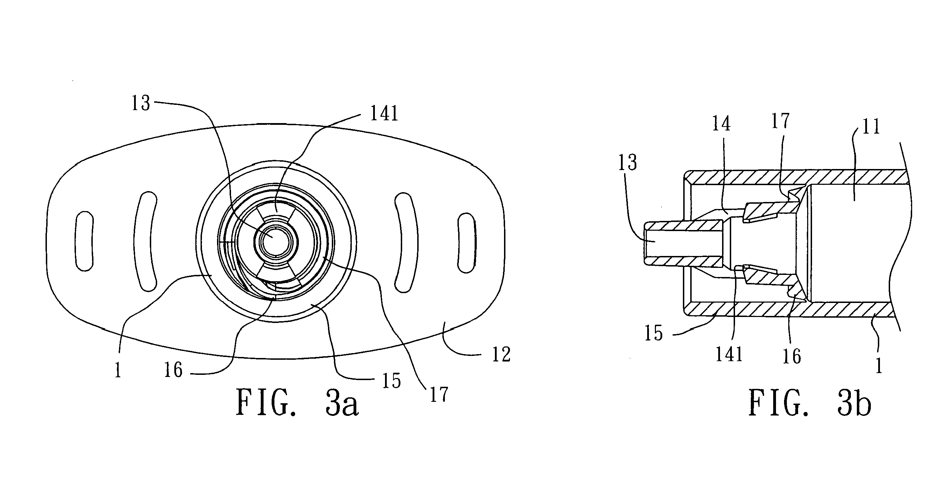

Referring to FIGS. 1a~4, a safety hypodermic syringe in accordance with the present invention is shown comprised of a barrel **1,** a needle assembly **2,** a plunger **3,** and a stopper **4.**

The barrel **1** comprises a fluid chamber **11** adapted to hold a liquid medicine, a finger flange **12** extending around the periphery of the rear end thereof for the holding of the hand, and a conical front connector **14** suspending inside the front end thereof. The conical front connector **14** defines an axially extending fluid passage **13** in communication between the fluid chamber **11** and the atmosphere, having two retaining portions, for example, two hollow retaining wings **141** protruded from the periphery at two sides, a connection portion **16** extending around the periphery of the bottom (inner) end thereof and connected to the peripheral wall **15** of the barrel **1**, and a tearing groove **17** formed on the connection portion **16.** When the user pushed the plunger **2** further forwards after the service of the safety hypodermic syringe (after injection), the connection portion **16** is broken along the tearing groove **17,** and therefore the conical front connector **14** is separated from the barrel **1.**

The barrel **1** further has an inside annular flange **18** extending around the inside wall thereof near the finger flange **12.**

The needle assembly **2** comprises a hub **22** and a needle cannula **21** fastened to the hub **22.** The hub **22** has an axial hole **23** disposed in fluid communication with the axial center through hole of the needle cannula **21,** and a rear coupling portion **24** capped on the conical front connector **14** of the barrel **1** and engaged into the tearing groove **17.** The rear coupling portion **24** has a beveled outer surface **241** disposed in contact with the connection portion **16.**

Because the rear coupling portion **24** is capped on the conical front connector **14** of the barrel 1 and engaged into the tearing groove **17** with the beveled outer surface **241** disposed in contact with the connection portion **16**, the needle assembly **2** is held positively in position and can stop leaking of the contained fluid medicine out of the barrel **1** in case the tearing groove **17** is broken accidentally. Therefore, the hub **22** of the needle assembly **2** has the functions of: (1) preventing biasing of the conical front connector **14** to cause breaking of the tearing groove **17** upon an external force. and (2) stopping the contained fluid medicine from leaking out of the barrel **1** when the tearing groove **17** is broken accidentally.

The plunger 3 comprises a radially ribbed shank **32**, a thumb rest **31** at the rear end of the shank **32,** a head **33** at the front end of the shank **32,** a neck **37** connected between the shank **32** and the head **33,** a front extension rod **34** axially forwardly extending from the center of the front side of the head **33,** a front tip **361** axially forwardly extending from the front end of the front extension rod **34,** a barbed retaining flange **36** extending around the periphery between the front extension rod **34** and the front tip **362,** and a stopper holder **35** formed integral with the front extension rod **34.** The stopper holder **35** comprises a front retaining skirt **351** and a rear retaining skirt **352** respectively extending around the periphery of the front extension rod **34** for holding the stopper **4.**

The stopper **4** is made out of a flexible material, for example, rubber. The outer diameter of the stopper **4** fits the diameter of the fluid chamber **11** of the barrel **1.** The stopper **4** has a longitudinal chamber **41,** which accommodates the front skirt **351** of the stopper holder **35** of the plunger **3** to secure the stopper **4** to the stopper holder **5,** allowing axial displacement of the stopper **4** relative to the stopper holder **5,** an inside annular flange **42** formed in the rear side of the longitudinal chamber **41** and disposed in contact with the periphery of the stopper holder **35** between the front skirt **351** and the rear skirt **352,** and a front center through hole **43** forwardly extending from the longitudinal chamber **41** to the front side at the center for the passing of the front extension rod **34** of the plunger **3.**

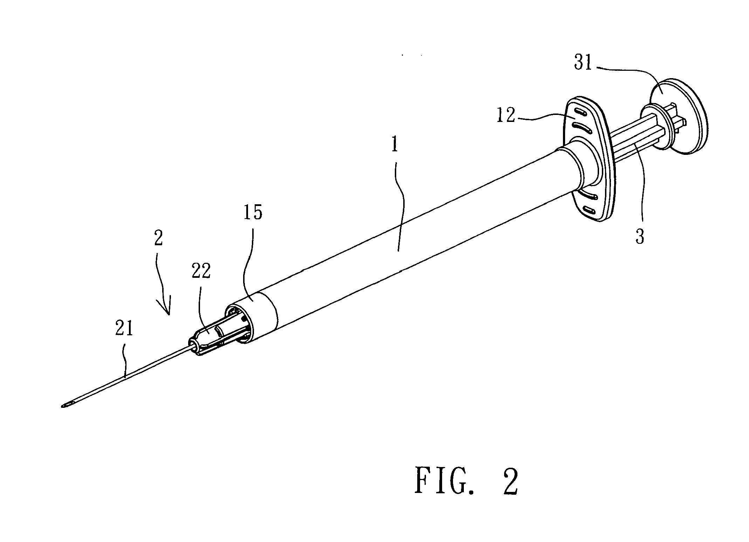

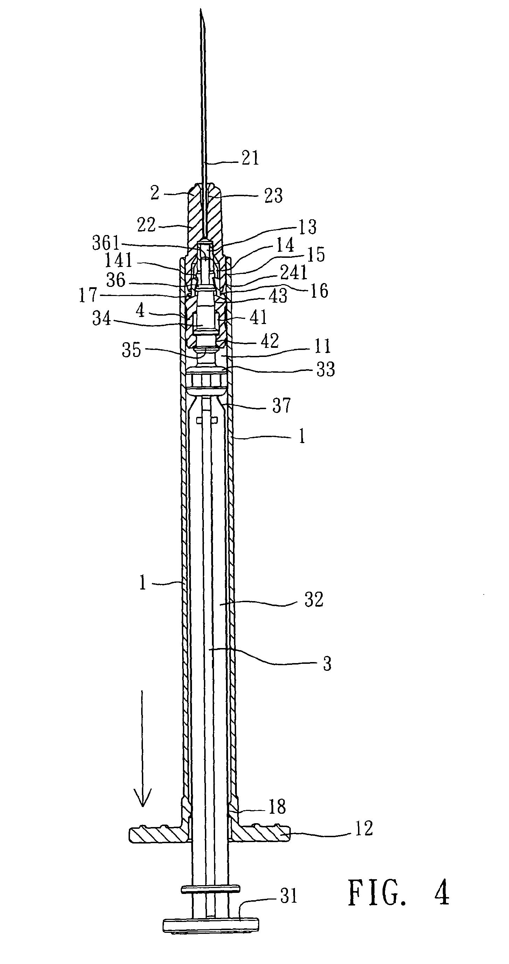

During the assembly process of the present invention, the stopper **4** is fastened to the front extension rod **34** of the plunger 3 to have the barbed retaining flange **36** stopped at the front side of the stopper **4** and the inside annular flange **42** be disposed in contact with the periphery of the stopper holder **35** between the front skirt **351** and rear skirt **352** of the stopper **4**, and then the stopper **4** is inserted with the plunger **3** into the fluid chamber **11** of the barrel **1** from the rear side, and then the rear coupling portion **24** of the hub **22** of the needle assembly **2** is capped on the conical front connector **14** of the barrel **1** and engaged into the tearing groove **17** with the beveled outer surface **241** disposed in contact with the connection portion **16.** FIGS. 2 and 4 show the safety hypodermic syringe assembled.

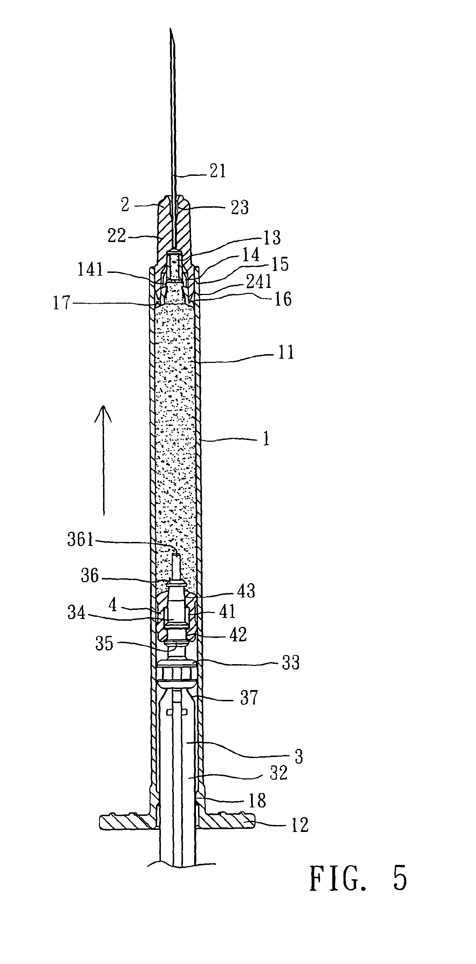

After the safety hypodermic syringe has sucked in the fluid medicine for injection, as shown in FIG. 5, push the plunger **3** to move the stopper **4** forwards along the fluid chamber **11** and to further squeeze the fluid medicine out of the conical front connector **14** and the needle assembly **2** into the patient's body.

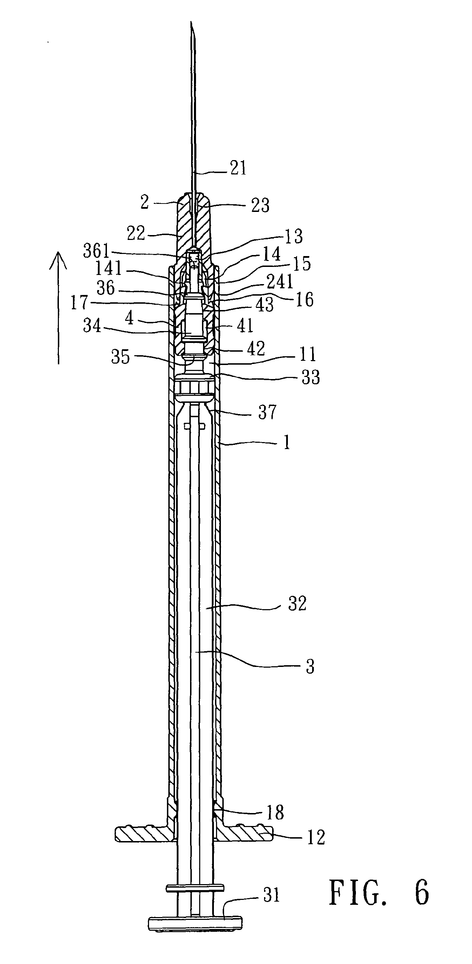

When the stopper **4** reached the connection portion **16** of the conical front connector **14** as shown in FIG. 6, the barbed retaining flange **36** of the plunger **3** is stopped at the rear end of the fluid passage **13** and the front tip **361** is inserted into the fluid passage **13** of the barrel **1**, expelling residual fluid medicine out of the conical front connector **14** of the barrel **1.**

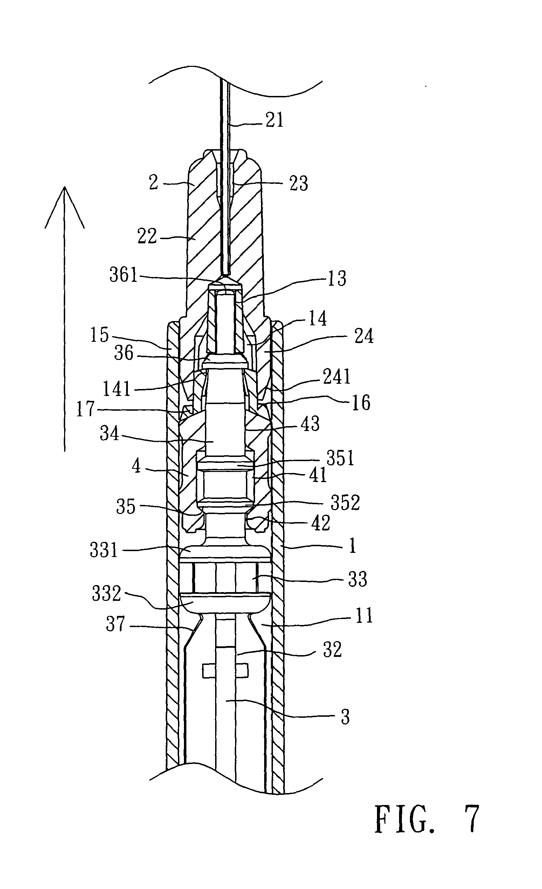

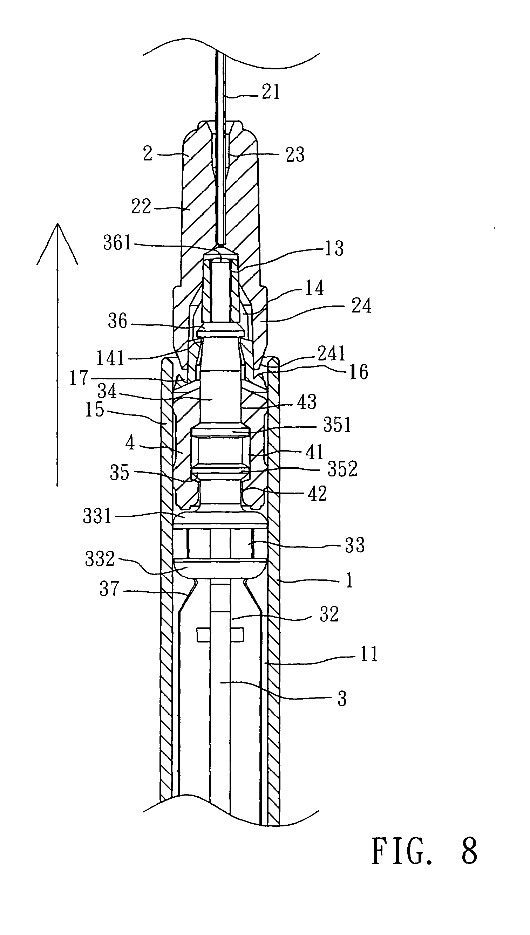

After the service of the safety hypodermic syringe as shown in FIG. 7, push the plunger **3** further forwards to force the barbed retaining flange **36** into engagement with the retaining wings **141** of the conical front connector **14.** Because the stopper **4** is stopped at the connection portion **16** of the conical front connector **14** at this time, pushing the plunger **3** further forwards causes forward displacement of the plunger **3** relative to the stopper 4 to move the rear skirt **352** over the inside annular flange **42** into the longitudinal open chamber **41** of the stopper **4** as shown in FIG. 8. When continuously pushing the plunger **3** forwards at this time, the tearing groove **17** will be forced to break, causing disconnection of the conical front connector **14** from the peripheral wall **15** of the barrel **1.** At this time, the broken safety hypodermic syringe becomes useless.

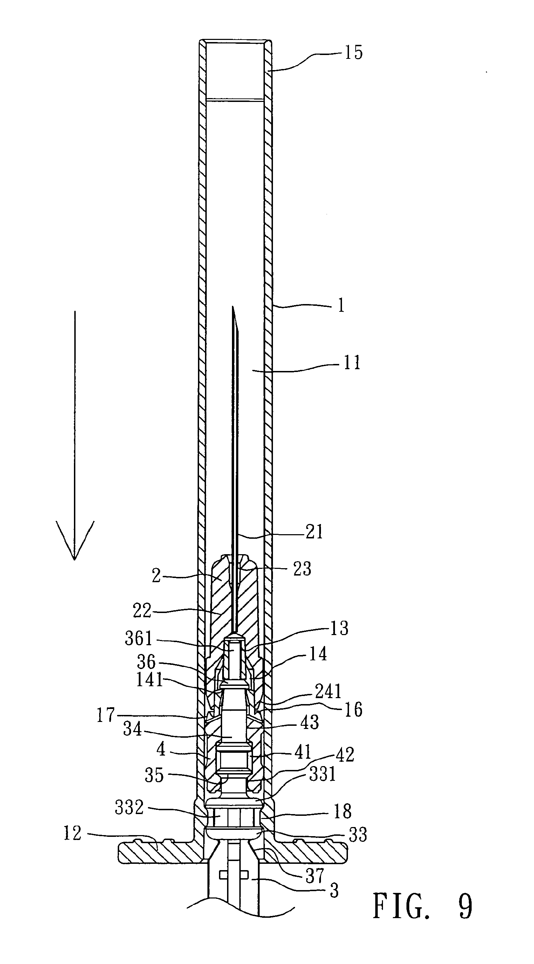

Thereafter, the plunger **3** is pulled backwards. At this time, as shown in FIG. 9, the stopper **4,** the broken conical front connector **14,** and the attached needle assembly **2** are carried with the plunger **3** into the inside of the fluid chamber **11** of the barrel **1** to have the front flange **331** and rear flange **332** of the head **3** be respectively stopped at the front and rear sides of the inside annular stop flange **18** of the barrel **1.**

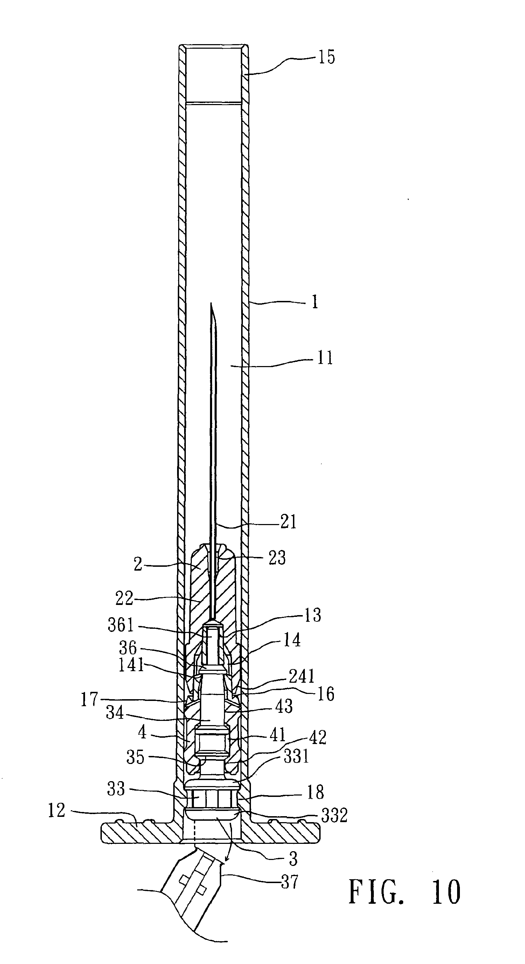

After the needle assembly **2** has been received inside the fluid chamber **11** of the barrel **1,** the user can bias the shank **32** against the inside wall of the barrel **1** to break the neck **37,** as shown in FIG. 10, keeping the needle assembly **2** and a part of the plunger **3** and the stopper **4** inside the fluid chamber **11** of the barrel **1.**

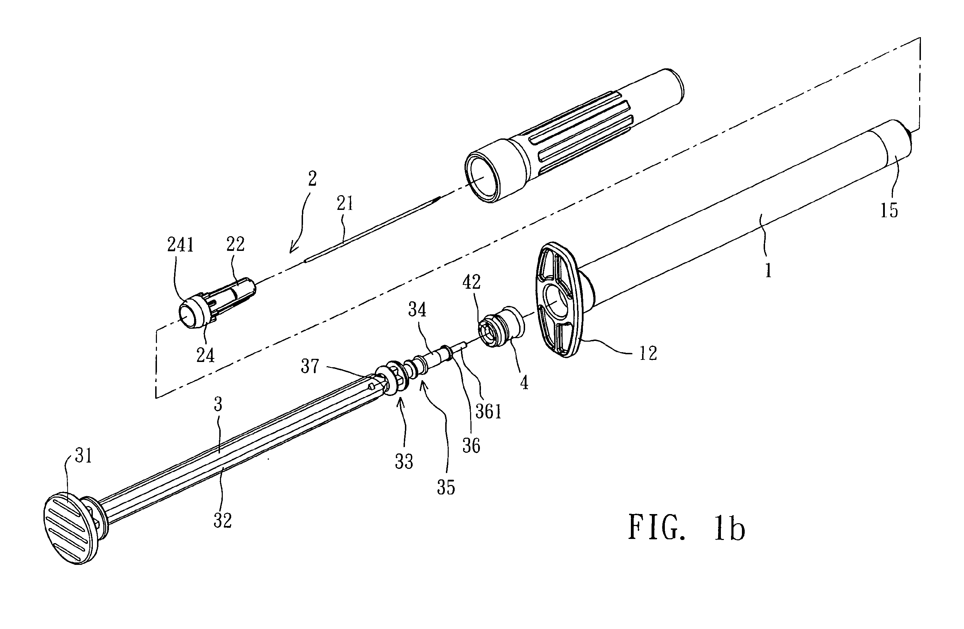

A prototype of safety hypodermic syringe has been constructed with the features of FIGS. 1~10. The safety hypodermic syringe functions smoothly to provide all the features discussed earlier.

Although a particular embodiment of the invention has been described in detail for purposes of illustration, various modifications and enhancements may be made without departing from the spirit and scope of the invention. Accordingly, the invention is not to be limited except as by the appended claims.

## Claims

1. A safety hypodermic syringe comprising:
a barrel, said barrel comprising a fluid chamber, and a front connector suspending in a front end thereof, said front connector defining an axially extending fluid passage in communication between said fluid chamber and the atmosphere, said front connector having two retaining portions protruded from the periphery thereof at two sides, a connection portion extending around the periphery of a rear end thereof and connected to the periphery of said barrel, and a tearing groove formed on said connection portion;
a needle assembly, said needle assembly comprising a hub and a needle cannula forwardly extending from said hub, said hub having an axial hole disposed in fluid communication with said needle cannula and a rear coupling portion capped on said front connector of said barrel and engaged into said tearing groove;
a plunger, said plunger comprising a shank, a front extension rod forwardly axially extending from a front end of said shank, and a retaining portion at said front extension rod; and
a flexible stopper fitting the diameter of said fluid chamber of said barrel, said stopper having a longitudinal chamber through which said front extension rod of said plunger passes, and a front center through hole forwardly extending from said longitudinal chamber to a front side thereof and stopped in front of the retaining portion of said plunger;
wherein when pushed said plunger forwards after the service of the safety hypodermic syringe, the retaining portion of said plunger is forced into engagement with the retaining portions of said front connector and said tearing groove is broken to separate said front connector from said barrel for enabling the broken front connector and said needle assembly to be pulled with said plunger backwards to the inside of said barrel upon a return stroke of said plunger at this time.

2. The safety hypodermic syringe as claimed in claim 1, wherein the retaining portions of said front connector are hollow retaining wings; the retaining portion of said plunger is a barbed retaining flange for engaging the hollow retaining wings of said front connector.

3. The safety hypodermic syringe as claimed in claim 1, wherein said barrel has an inside annular stop flange extending around an inside wall near a rear end thereof; said plunger has a head connected between said shank and said front extension rod, said head having a front flange and a rear flange for engagement with the inside annular stop flange of said barrel after a return stroke of said plunger after the service of the safety hypodermic syringe.

4. The safety hypodermic syringe as claimed in claim 3, wherein said plunger further has a neck connected between said shank and said head for breaking to separate said shank from said head after engagement of the front flange and rear flange of said head with the inside annular stop flange of said barrel.

5. The safety hypodermic syringe as claimed in claim 1, wherein the coupling portion of said hub of said needle assembly has a beveled outer surface disposed in contact with said connection portion of said front connector of said barrel.

6. The safety hypodermic syringe as claimed in claim 1, wherein said plunger further comprises a stopper holder formed integral with said front extension rod for holding said stopper, said stopper holder having a front retaining skirt and a rear retaining skirt respectively extending around the periphery thereof; said flexible stopper having an inside annular flange formed in a rear side of said longitudinal chamber and disposed in contact with the periphery of said stopper holder between said front skirt and said rear skirt.

7. A safety hypodermic syringe comprising:
a barrel, said barrel comprising a fluid chamber, and a front connector suspending in a front end thereof, said front connector defining an axially extending fluid passage in communication between said fluid chamber and the atmosphere, said front connector having two hollow retaining wings protruded from the periphery thereof at two sides, a connection portion extending around the periphery of a rear end thereof and connected to the periphery of said barrel, and a tearing groove formed on said connection portion;
a needle assembly, said needle assembly comprising a hub and a needle cannula forwardly extending from said hub, said hub having an axial hole disposed in fluid communication with said needle cannula and connected to said front connector of said barrel;
a plunger, said plunger comprising a shank, a front extension rod forwardly axially extending from a front end of said shank, and a barbed retaining flange around the periphery of a front end of said front extension rod; and
a flexible stopper fitting the diameter of said fluid chamber of said barrel, said stopper having a longitudinal chamber through which said front extension rod of said plunger passes, and a front center through hole forwardly extending from said longitudinal chamber to a front side thereof and stopped in front of the barbed retaining flange of said plunger;
wherein when pushed said plunger forwards after the service of the safety hypodermic syringe, the barbed retaining flange of said plunger is forced into engagement with the hollow retaining wings of said front connector and said tearing groove is broken to separate said front connector from said barrel for enabling the broken front connector and said needle assembly to be pulled with said plunger backwards to the inside of said barrel upon a return stroke of said plunger at this time.

8. The safety hypodermic syringe as claimed in claim 7, wherein said barrel has an inside annular stop flange extending around an inside wall near a rear end thereof; said plunger has a head connected between said shank and said front extension rod, said head having a front flange and a rear flange for engagement with the inside annular stop flange of said barrel after a return stroke of said plunger after the service of the safety hypodermic syringe.

9. The safety hypodermic syringe as claimed in claim 8, wherein said plunger further has a neck connected between said shank and said head for breaking to separate said shank from said head after engagement of the front flange and rear flange of said head with the inside annular stop flange of said barrel.

10. The safety hypodermic syringe as claimed in claim 7, wherein said plunger further comprises a stopper holder formed integral with said front extension rod for holding said stopper, said stopper holder having a front retaining skirt and a rear retaining skirt respectively extending around the periphery thereof; said flexible stopper having an inside annular flange formed in a rear side of said longitudinal chamber and disposed in contact with the periphery of said stopper holder between said front skirt and said rear skirt.
